# FASCICULE DE BREVET EUROPEEN

(11) **EP 1 334 205 B1**
(45) Date de publication et mention de la délivrance du brevet: **26.10.2005**
(21) Numéro de dépôt: 01996621.7
(22) Date de dépôt: 19.11.2001
(51) Int. Cl.: C12Q 1/04, C12Q 1/44, C12Q 1/37, C12Q 1/42, C12N 1/20

(54) **PROCEDE D'IDENTIFICATION DE LISTERIA MONOCYTOGENES ET MILIEU DE CULTURE**
VERFAHREN ZUR IDENTIFIZIERUNG VON LISTERIA-MONOZYTOGENEN UND KULTURMEDIUM
METHOD FOR IDENTIFYING LISTERIA MONOCYTOGENES AND CULTURE MEDIUM

(30) Priorité: 17.11.2000 FR 0014892
(43) Date de publication de la demande: 13.08.2003
(73) Titulaire: Biomerieux S.A., 69280 Marcy l'Etoile (FR)
(72) Inventeur: ROGER-DALBERT, Céline, F-01150 Vaux en Bugey (FR); BARBAUX, Laurence, F-01500 Amberieu-En-Bugey (FR)
(74) Mandataire: Bonneau, Gérard
(86) Numéro de dépôt international: PCT/FR2001/003627
(87) Numéro de publication internationale: WO 2002/040705

(56) Documents cités:
- WO-A-99/04032
- FR-A- 2 697 028
- US-A- 5 364 767
- US-A- 5 962 251
- BARCLAY R ET AL: "HAEMOLYSINS AND EXTRACELLULAR ENZYMES OF LISTERIA-MONOCYTOGENES AND LISTERIA-IVANOVII" JOURNAL OF MEDICAL MICROBIOLOGY, vol. 30, no. 2, 1989, pages 111-118, XP001028569 ISSN: 0022-2615
- KEROUANTON A ET AL: "Comparison of five typing methods for the epidemiological study of Listeria monocytogenes." INTERNATIONAL JOURNAL OF FOOD MICROBIOLOGY, vol. 43, no. 1-2, 18 août 1998 (1998-08-18), pages 61-71, XP001028066 ISSN: 0168-1605
- COX L J ET AL: "ENHANCED HEMOLYSIS AGAR EHA AN IMPROVED SELECTIVE AND DIFFERENTIAL MEDIUM FOR ISOLATION OF LISTERIA-MONOCYTOGENES" FOOD MICROBIOLOGY (LONDON), vol. 8, no. 1, 1991, pages 37-50, XP001030164 ISSN: 0740-0020
- MOBASHERY S: "Conscripting beta-Lactamase for Use in Drug Delivery. Synthesis and Biological Activity of a Cephalosporin C10-Ester of an Antibiotic Dipeptide" JOURNAL OF THE AMERICAN CHEMICAL SOCIETY, vol. 108, 1986, pages 1685-1686, XP002179509
- GROVES R D ET AL: "SEPARATION OF PATHOGENIC FROM APATHOGENIC LISTERIA-MONOCYTOGENES BY 3 IN-VITRO REACTIONS" JOURNAL OF CLINICAL MICROBIOLOGY, vol. 5, no. 6, 1977, pages 559-563, XP001030471 EN ISSN: 0095-1137

## Description

La présente invention concerne un substrat chromogène permettant l'identification directe de bactéries pathogènes du genre *Listeria*, et plus précisément de l'espèce *Listeria monocytogenes*.

L'invention concerne également l'association de deux substrats, l'un sensiblement spécifique de l'espèce *Listeria monocytogenes* et l'autre spécifique ou non du genre *Listeria*. Elle concerne également un milieu de culture contenant un tel substrat ou une telle combinaison de substrats. Elle concerne enfin un procédé d'identification utilisant de tels milieux de culture.

Depuis de très nombreuses années, on utilise des substrats particuliers pour permettre la détermination de la présence ou de l'absence d'activités enzymatiques caractéristiques de micro-organismes. Par le choix des substrats, selon qu'il y a réaction ou non, il est possible de caractériser la nature d'un groupe de micro-organismes ou de différencier des souches et/ou des espèces d'un genre microbien donné.

Les substrats synthétiques d'enzymes, tels que ceux utilisés dans la présente invention, sont constitués de deux parties, une première partie spécifique de l'activité enzymatique à révéler, ci-après appelée partie cible, et une seconde partie faisant office de marqueur, ci-après appelée partie marqueur.

Ces substrats particuliers peuvent être fluorescents ou chromogènes. En fait, c'est la seconde partie marqueur ou le produit de sa réaction avec un ou plusieurs autres composés, voir à ce sujet la demande de brevet PCT/FR99/00781 déposée au nom de la demanderesse, qui est fluorescente ou chromogène, lorsqu'elle n'est plus associée à la première partie cible.

L'isolement et l'identification de la bactérie *Listeria monocytogenes* est un problème majeur de la surveillance de l'hygiène agro-alimentaire et de la bactériologie médicale. Parmi les bactéries du genre *Listeria* seule l'espèce *Listeria monocytogenes* est connue pour être pathogène pour l'homme. Elle peut engendrer la listériose, parfois mortelle (25 à 30 % des cas) chez les personnes immunodéprimées, les enfants en bas âge ou les femmes enceintes. Les autres espèces de *Listeria* ne sont pas pathogènes ou ne le sont que pour les animaux. C'est le cas notamment de *Listeria ivanovii*.

Même si les risques de listériose humaine ont régressé dans la plupart des pays développés lors des dernières décennies, la société moderne exige de plus en plus de sécurité qui, si elle s'accommode de cas sporadiques, ne peut tolérer les épidémies.

Or en France par exemple, la politique de maîtrise des risques privilégie l'aval (taux de contamination dans les produits transformés) à l'amont (contamination des élevages). De ce fait, entre 15 et 60 % des carcasses de volailles, 3 et 36 % des carcasses de porcs et 7 et 28 % des carcasses de bovins seraient contaminées par les *Listeria*.

Dans le cadre de diagnostic d'affections bactériennes chez l'humain, il est donc important de distinguer nettement *Listeria monocytogenes* parmi les autres bactéries du genre *Listeria spp*. qui ne sont pas pathogènes.

*La détection et l'isolement de* Listeria spp. *sont classiquement réalisés avec des milieux de culture sélectifs. Les milieux sélectifs Palcam (Van Netten* et al., *J. Food Microbiol. (1988),* *6**, pp. 187-188) et Oxford (Curtis* et al., *Lett. Appl. Microbiol. (1989),* *8**, pp. 85-98) sont les plus couramment utilisés. Ces milieux permettent la détection de toutes les espèces du genre* Listeria spp. *Ainsi les colonies typiques observées doivent ensuite être soumises à des tests d'identification supplémentaires, tels que des tests microscopiques et*/*ou biochimiques et*/*ou immunologiques et*/*ou génétiques, de manière à vérifier l'appartenance à l'espèce* Listeria monocytogenes.

Or, ces manipulations supplémentaires augmentent la durée et le coût des analyses. Elles nécessitent une multitude de réactifs et l'intervention de personnel qualifié. De plus, le prélèvement des colonies soumises à l'identification étant aléatoire, les manipulations supplémentaires sont souvent source d'erreur ou au moins, la cause d'une moindre précision et fiabilité des résultats. C'est le cas notamment, lorsque sur le milieu d'isolement, les colonies de *Listeria monocytogenes* sont très minoritaires par rapport aux autres colonies formées par les autres espèces de *Listeria.*

Le brevet EP-B-0.496.680, délivré à la demanderesse, décrit un procédé d'analyse bactériologique pour différencier l'espèce *Listeria monocytogenes* des autres bactéries du genre Listeria. Selon ce procédé, on utilise un milieu d'identification comprenant un substrat chromogène ou fluorogène susceptible d'être hydrolysé par une enzyme appelée Glycine aminopeptidase. Le milieu utilisé peut également contenir éventuellement un substrat de fermentation et/ou un substrat réductible et/ou un substrat hydrolysable par voie enzymatique, tel que le substrat de l'α-mannosidase, dont la transformation chimique permet de caractériser l'espèce *Listeria* présente dans l'échantillon à analyser.

Cette technique très intéressante présente un inconvénient majeur résidant dans le fait que l'espèce *Listeria monocytogenes* est la seule à ne pas avoir d'activité enzymatique Glycine aminopeptidase. Il est donc possible de détecter tout le genre *Listeria*, à l'exception de l'espèce *Listeria monocytogenes*, qui est pathogène. La détection de *Listeria monocytogenes* par une activité négative n'est donc pas aisée, l'utilisation d'un test négatif manquant de spécificité en cas de mutant ou si les autres espèces sont stressées et ne répondent pas avec une activité normale.

L'article « *Separation of pathogenic and apathogenic listeria-monocytogenes by 3 in-vitro réaction* », Groves, R D et al., Journal of Clinical Microbiology, vol 5, no. 6, 1977, décrit l'utilisation de substances qui sont activées du fait de leur clivage par une enzyme spécifique, mais non l'utilisation de substrats d'estérases.

L'article *« Enhance haemolysis agar EHA an improved selective and differential medium for isolation of Listeria monocytogenes* », Cox L J et al., Food Microbiology (London), Vol. 8, No. 1, 1998, qui concerne une méthode permettant d'isoler les bactéries du genre *Listeria monocytogenes*, ne décrit ni ne suggère l'utilisation de substrats d'estérases.

Le document FR 2 687 028 décrit un milieu de culture destiné à la mise en évidence de bactéries du genre *Salmonella* et non *Listeria* monocytogenes.

L'article « *comparison of five typing methods for the epidemiological study of Listeria Monocytogenes* », Kerouaton et al., International Journal of Food Microbiology, vol 43, No. 1-2, décrit des méthodes permettant de discriminer entre plusieurs souches de *Listeria monocytogenes* mais qui ne permettent pas d'identifier spécifiquement la présence de *Listeria monocytogenes*.

Dans l'article « Haemolysins and extra cellular enzymes of listeria-monocytogenes and listeria-Ivanovii », Barclay R et al., Journal of Medical Microbiology, Vol 30, No.2, 1989, il est décrit l'utilisation de substrats d'estérases pour détecter des bactéries du genre listeria mais ne permet pas de distinguer entre *Listeria monocytogenes* et *Listeria Ivanovii*.

De plus, il est connu d'utiliser des milieux chromogéniques permettant de détecter l'activité β-glucosidase spécifique du genre *Listeria*.

Ainsi, les brevets des Etats-Unis No. 5,962,251 et US 5,364,767 décrivent une méthode pour l'identification de microorganismes comprenant deux chromogènes mais ne décrit pas l'utilisation de substrat d'estérases pour l'identification de *Listeria monocytogenes*.

Par ailleurs, pour une discrimination plus précise utilisant également des milieux chromogéniques, il est possible de différencier les espèces *Listeria monocytogenes* et *Listeria ivanovii*, des autres Listeria par la mise en évidence de l'activité phospholipase C spécifique du phosphatidylinositol (PI-PLC).

En effet, il a été démontré que la PI-PLC est sécrétée dans le milieu de culture par certaines espèces du genre *Listeria* telles que *Listeria monocytogenes* et *Listeria ivanovii* (Leimeister-Wächter et al., Mol. Microbiol. (1991) 5(2), pp. 361-366 ; J. Mengaud et al., Mol. Microbiol. (1991) 5(2), pp. 367-372 ; et Goldfine et al., Infection and Immunity (1992) 60(10), pp. 4059-4067). On sait également qu'il est possible *d'identifier ces deux espèces grâce à des procédés indirects (Notermans* et al., *App. and Env. Microbiology (1991),* *vol. 57 n°9,* *pp. 2666-2670*.

*La demande de brevet WO-A-99*/*04032 décrit un milieu de culture contenant un substrat chromogène spécifique de* Listeria monocytogenes *et de* Listeria ivanovii, *il s'agit d'un dérivé du Phosphatidylinositol, tel que le sel d'ammonium du 5-Bromo-4-chloro-3-indolyl-myo-inositol-1-phosphate, permettant une détection directe de ces deux espèces, c'est-à-dire en une étape, et donc leur différenciation par rapport aux autres espèces de* Listeria.

*L'utilisation de la PI-PLC pour la détection bactériologique est également reprise dans les documents suivants :*
- *WO-A-98*/*38332, qui concerne essentiellement une méthode et un test de détection de la PI-PLC par l'intermédiaire d'un substrat clivé par une telle enzyme, un des résidus du substrat étant chromogène et permettant l'identification de* Listeria *pathogènes, et*
- *WO99*/*48899, qui propose un composé fluorogène à base de 4-Méthylumbelliférone permettant la détection d'une activité enzymatique, la PI-PLC, présente chez de nombreuses espèces* Clostridium species, Listeria ivanovii, Staphylococcus aureus, Bacillus cereus, Bacillus thuringiensis *et également* Listeria monocytogenes.

Ce dernier document met en exergue le fait que l'activité enzymatique phospholipase C n'est pas spécifique de *Listeria monocytogenes* puisqu'elle est également présente chez d'autres espèces, dont une autre *Listeria*, c'est-à-dire *Listeria ivanovii*.

Cependant, il faut noter que les substrats chromogéniques d'estérase sont plus simples à synthétiser et moins chers que ceux de PI-PLC. De plus, il est avantageux d'utiliser un substrat chromogénique plutôt qu'un substrat naturel, car la détection à l'oeil nu est plus facile : mise en évidence de colonies colorées au lieu de la révélation d'un halo autour des colonies. Cet avantage se retrouve lors de cultures plurimicrobiennes : chacune des colonies ayant sa couleur spécifique alors que le halo peut s'étendre sous deux colonies différentes.

La présente invention a pour objectif de proposer une méthode de détection qui permettent la différenciation de l'espèce *Listeria monocytogenes* par rapport à toutes les autres espèces de *Listeria*. Ce résultat est obtenu par détection d'au moins une activité métabolique, qui n'était pas utilisée jusqu'à présent pour la détection des *Listeria* et de la différenciation des *Listeria monocytogenes* par rapport aux autres espèces du genres *Listeria.* Il s'agit d'une activité estérase qui est fortement exprimée par *Listeria monocytogenes* et très faiblement par les autres espèces du même genre, et d'autre part, au moins une activité présente chez toutes ou partie des Listeria, telle que osidase ou phosphatase ou aminopeptidase, qui permet d'augmenter le contraste de coloration entre *Listeria monocytogenes* et les autres espèces du même genre. Il est ainsi possible de séparer *Listeria monocytogenes* par rapport *à Listeria ivanovii* et *Listeria innocua*, qui sont les espèces les plus fréquemment isolées et dont les caractéristiques enzymatiques sont très proches de celles de *Listeria monocytogenes*.

A cet effet, la présente invention concerne l'utilisation d'un substrat pour l'identification directe de bactéries pathogènes *Listeria monocytogenes*, qui se caractérise par le fait que le substrat est clivé par une activité estérase, différente de l'activité phospholipase C, spécifique du phosphatidylinositol (PI-PLC), activité estérase qui est spécifique de *Listeria monocytogenes*, et en ce qu'il comprend une partie cible constituée par un acide gras ayant une chaîne carbonée de longueur comprise entre 4 et 20 atomes de carbones.

Ainsi si l'activité PI-PLC est une activité estérase, *Listeria ivanovii* est estérase négative alors qu'elle est PI-PLC positive et peut donc être confondue avec *Listeria monocytogenes*. Contrairement à l'état de la technique et à la détection de la PI-PLC qui ne permet pas de différencier *Listeria monocytogenes* par rapport à d'autres *Listeria,* la présente invention permet une détection plus spécifique de *Listeria monocytogenes*.

Selon un mode préférentiel de réalisation, l'activité estérase est une activité enzymatique spécifique, c'est-à-dire qui clive, la liaison ester entre la partie marqueur et la partie cible qui constituent le substrat.

Selon un mode préférentiel de réalisation, la liaison clivée est une liaison ester entre une fonction alcool portée par la partie marqueur, et un acide organique constituant la partie cible.

Selon un mode préférentiel de réalisation, la partie marqueur est constituée par un chromogène, tel qu'un Indoxyle pouvant être constitué par l'un des constituants suivants :
- 5-Bromo-3-indoxyle, ou
- 5-Bromo-4-chloro-3-indoxyle, ou
- 6-Chloro-3-indoxyle, ou
- 5-Bromo-6-chloro-3-indoxyle, ou
- 6-Bromo-3-indoxyle.

Selon un mode préférentiel de réalisation, la partie cible est constitué par un acide gras ayant une chaîne carbonée de longueur comprise entre 4 et 10 atomes de carbones.

Préférentiellement, le substrat est constitué par le 5-Bromo-4-chloro-3-indolyl butyrate, le 5-Bromo-4-chloro-3-indolyl octanoate, 5-Bromo-4-chloro-3-indolyl nonanoate ou le 5-Bromo-4-chloro-3-indolyl décanoate.

Selon une variante de réalisation, le substrat est couplé à au moins un autre substrat permettant de détecter au moins une autre activité enzymatique possédée par toute ou partie des espèces de *Listeria*.

Dans le cas où il y a deux substrats, le substrat permettant de détecter l'activité estérase, à l'exception de l'enzyme PI-PLC, a une coloration différente de celle de l'autre substrat permettant de détecter une activité enzymatique différente de l'activité estérase précédemment définie.

Selon un mode préférentiel de réalisation, l'autre activité enzymatique possédée par toute ou partie des espèces de *Listeria* est une activité osidase, phosphatase ou aminopeptidase.

Préférentiellement, la partie marqueur de l'autre substrat est à base de :
- 5-Bromo-3-indoxyle, ou
- 5-Bromo-4-chloro-3-indoxyle, ou
- 6-Chloro-3-indoxyle, ou
- 5-Bromo-6-chloro-3-indoxyle, ou
- 6-Bromo-3-indoxyle.
En ce qui concerne le 6-Chloro-3-indoxyle, une telle molécule est particulièrement bien décrite dans le brevet US-A-5,364,767, où elle est essentiellement associée au N-Acetyl-β-D-galactosaminide, au N-Acetyl-β-D-glucosaminide, au Butyrate, à l'Octanoate, au sel de Phosphate p-toluidine, au Sulfate ou au β-D-Glucopyranoside. Elle peut être à la base également de 5-Bromo-6-chloro-3-indoxyle.

Selon une variante de réalisation, l'autre substrat est constitué par :
- 5-Bromo-6-chloro-3-indolyl-β-D-glucoside, ou
- 6-Chloro-3-indolyl-β-D-glucoside, ou
- 6-Chloro-3-indolyl-β-D-cellobioside, ou
- 6-Chloro-3-indolyl-N-acétyl-β-D-glucosaminide, ou
- 6-Chloro-3-indolyl-α-D-mannoside, ou
- 6-Chloro-3-indolylphosphate.

La présente invention concerne également l'utilisation d'un milieu réactionnel, pour l'identification directe de *Listeria monocytogenes*, qui utilise au moins un substrat, tel que défini ci-dessus.

Plus précisément, le substrat permettant de détecter l'activité estérase, différente de l'activité PI-PLC, a une concentration comprise entre 20 mg/l et 3 g/l, ou préférentiellement entre 50 mg/l et 1 g/l, ou préférentiellement entre 100 et 600 mg/l, ou environ de 250 mg/l.

Plus précisément, le milieu comprend au moins au autre substrat permettant de détecter une autre activité, telle qu'une activité osidase, phosphatase ou aminopeptidase, a une concentration comprise entre 10 mg/l et 500 mg/l, préférentiellement entre 50 et 300 mg/l, et encore plus préférentiellement entre 100 et 200 mg/l.

Le milieu est de préférence un milieu gélosé ou liquide.

Selon une variante de réalisation, le milieu comporte un moyen permettant de discriminer *Listeria monocytogenes* par rapport à *Listeria welshimeri* et *Listeria seeligeri,* à savoir :
- ajout d'au moins un Hydrate de carbone acidifié (par exemple Xylose et/ou D-Tagatose) par *Listeria welshimeri* et *Listeria seeligeri* et pas par *Listeria monocytogenes*,
- ajout d'au moins un Hydrate de carbone acidifié par *Listeria monocytogenes* et éventuellement *Listeria innocua* et/ou *Listeria ivanovii* et/ou *Listeria grayii*,
- ajout d'au moins un substrat permettant de révéler une activité osidase (β-maltosidase) et/ou phosphatase et/ou aminopeptidase (L-glycine aminopeptidase) spécifique(s) au moins de *Listeria welshimeri* et *Listeria seeligeri* mais pas de *Listeria monocytogenes*, ou
- ajout d'au moins un substrat naturel de Phospholipase (PLC), tel que Phosphatidylinositol (PI) et/ou Phosphatidylcholine (PC).

Selon un mode particulier de réalisation, le milieu comporte un moyen sélectif permettant de discriminer *Listeria monocytogenes* par rapport à au moins les espèces suivantes :
- *Staphylococcus aureus*,
- *Bacillus thuringiensis*,
- *Enterococcus faecalis*,
- *Escherichia coli,*
- *Pseudomonas aeruginosa*, et
- *Candida albicans*.

Selon un mode préférentiel de réalisation, le moyen sélectif est constitué par au moins l'un des composés suivants :
- Chlorure de Lithium,
- Ceftazidime,
- Amphotéricine B,
- Fosfomycine, et/ou
- Colistine.

La présente invention concerne encore un procédé d'identification de bactéries pathogènes de l'espèce *Listeria monocytogenes*, comprenant :
- l'ensemencement d'un échantillon susceptible de contenir les bactéries pathogènes sur un milieu de culture, tel que défini ci-dessus,
- l'incubation du milieu de culture ensemencé avec l'échantillon, et
- la détermination de la présence desdites bactéries pathogènes par la couleur et l'intensité caractéristiques du ou des substrats.

Selon une variante de réalisation, ledit échantillon, susceptible de contenir des bactéries pathogènes est préalablement enrichi avant d'être ensemencé sur un milieu de culture, tel que défini ci-dessus.

Selon une autre variante de réalisation du procédé, on effectue la détermination de la présence desdites bactéries pathogènes, par la couleur et l'intensité caractéristiques du ou des substrats, après 18 à 24 heures d'incubation.

Enfin, l'invention propose une utilisation d'un substrat constitué d'une partie cible, telle que définie ci-dessus, et d'une partie inhibitrice pour inhiber spécifiquement *Listeria monocytogenes* lorsque la partie inhibitrice est libérée.

La présente invention concerne donc essentiellement l'identification de l'espèce *Listeria monocytogenes* pathogène pour l'homme, par rapport aux autres espèces du genre *Listeria*.

L'activité enzymatique estérase est un bon identifiant de l'espèce *Listeria monocytogenes* par rapport aux autres espèces du genre *Listeria*, à l'exception de l'activité estérase plus spécifique dite PI-PLC.

Ainsi, l'état de la technique décrit précédemment met en évidence le manque de spécificité de la PI-PLC, qui est spécifique d'un type de lipides, entre les espèces *Listeria monocytogenes* et *Listeria ivanovii*, par exemple. La PI-PLC hydrolyse les dérivés de Phosphatidylinositol entre le Glycérol et le Phosphate inorganique. Dans le cas d'un substrat chromogénique, l'enzyme coupe entre le marqueur et le Phosphate inorganique lié à l'Inositol, comme décrit ci-dessous.

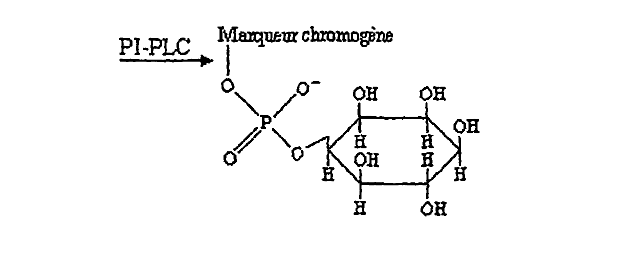

Les estérases, utilisées dans la présente invention, hydrolysent les lipides contenant un ou plusieurs acides gras, dont la longueur de chaîne est préférentiellement comprise entre 7 et 10 atomes de carbone. Ces estérases hydrolysent les liaisons esters entre un alcool et un acide gras organique, comme décrit ci-dessous.

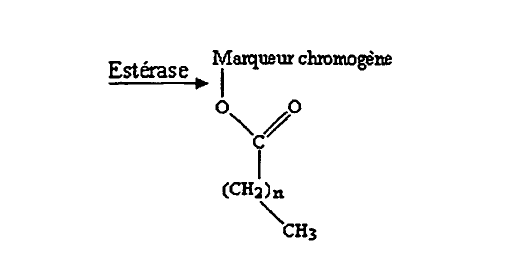

### Expérience 1 : Test de plusieurs substrats d'estérase possédant le même marqueur chromogène mais de différentes longueurs de chaîne d'acides gras :

Les substrats chromogéniques d'estérase testés sont :
- le 5-Bromo-3-indolyl octanoate, ci-après référencé Blue-C8,
- le 5-Bromo-3-indolyl nonanoate, ci-après référencé Blue-C9, et
- le 5-Bromo-3-indolyl décanoate, ci-après référencé Blue-C10.

Une solution mère de chaque substrat à 40 g/l est réalisée dans un mélange 40 % Diméthylsulfoxide et 60 % Polyoxyéthylènesorbitan Monolaurate (Tween 20). Un volume suffisant est ajouté à trois milieux type Columbia en surfusion pour obtenir une concentration finale en substrat de 250 mg/l. Des micro-organismes issus de la collection de la demanderesse ou de la collection ATCC ont été ensemencés sur ce milieu par isolement en trois cadrans à partir d'une suspension à 0,5 McFarland. Les boîtes ont été incubées à 37°C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 1 ci-dessous :

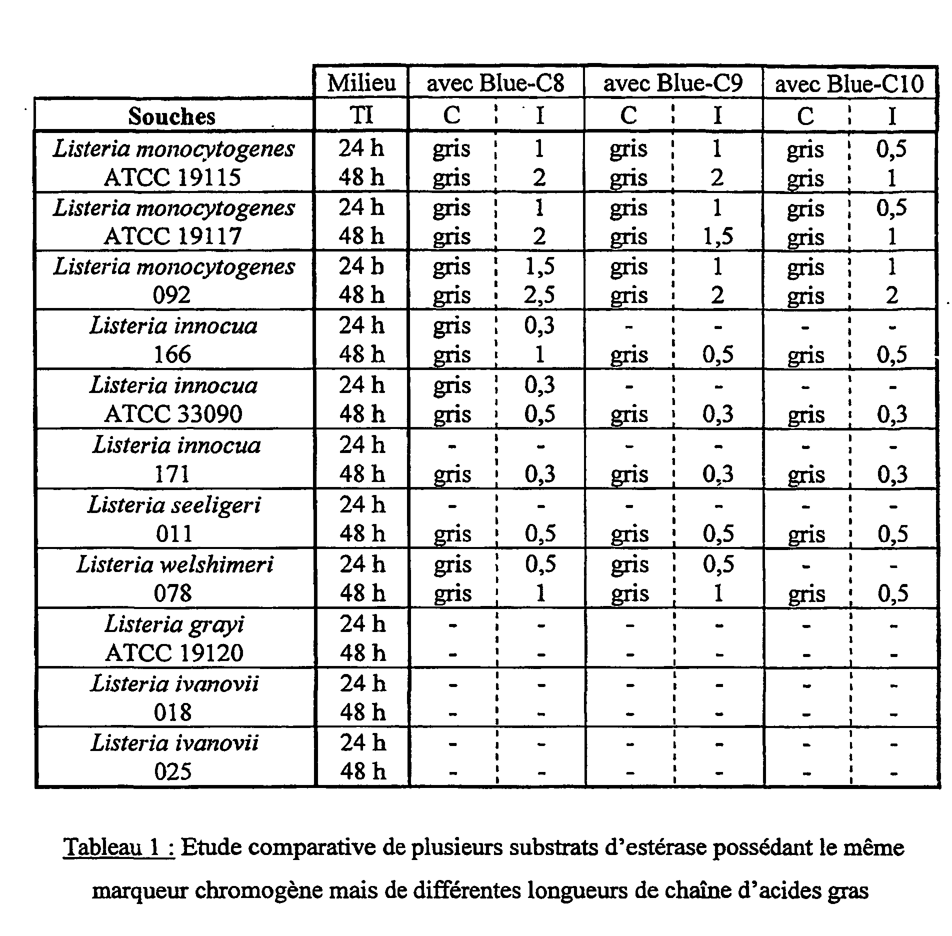

Dans le tableau 1 ci-dessus comme dans les tableaux qui suivront, C représente la coloration des colonies après incubation, I représente l'Intensité de cette coloration, le symbole « - » est synonyme d'une absence de couleur ou d'intensité, enfin TI définit les temps d'incubation. A noter que l'intensité de la coloration est une échelle arbitraire mais qui est commune à l'ensemble des échantillons biologiques et des milieux testés. Cette échelle est valable pour cette expérience ainsi que pour toutes les expériences qui suivront. Elle peut être définie de la manière suivante :
- 0 correspond à une absence d'activité,
- 0,1 correspond à la présence d'une trace de coloration,
- 0,5 correspond à la présence d'une coloration très pâle,
- 1 correspond à la présence d'une coloration nette de faible intensité,
- 1,5 correspond à la présence d'une coloration intermédiaire aux colorations 1 et 2,
- 2 correspond à la présence d'une coloration franche d'intensité moyenne,
- 2,5 correspond à la présence d'une coloration intermédiaire aux colorations 2 et 3,
- 3 correspond à la présence d'une coloration intense,
- 3,5 correspond à la présence d'une coloration intermédiaire aux colorations 3 et 4,
- 4 correspond à la présence d'une coloration très intense.

Seules les souches de *L. monocytogenes, L. innocua, L. seeligeri et L. welshimeri* présentent une coloration grise, ces souches expriment donc une activité estérase. Il est possible de remarquer, quelle que soit la longueur de chaîne de l'acide gras étudiée, qu'il y a, en terme d'intensité, un écart d'environ 1 unité entre les souches de *L. monocytogenes* qui présentent les intensités maximales et les souches de *L. innocua, L. welshimeri et L. seeligeri* qui présentent les intensités minimales.

Toutefois, pour la suite des exemples seuls les substrats dérivés d'octanoate et de nonanoate seront étudiés.

De plus, on peut observer que seules les souches de *Listeria monocytogenes* présentent une coloration à 24 heures. Il est donc possible de différencier *Listeria monocytogenes* d'autres espèces de *Listeria* en fonction de la température d'incubation.

### Expérience 2 : Test de plusieurs substrats d'estérase possédant différents marqueurs chromogènes à base d'Indoxyl et ayant comme acides gras l'Acide octanoïque :

Les substrats chromogéniques d'estérase testés sont :
- le 5-Bromo-3-indolyl octanoate, ci-après référencé Blue-C8,
- le 5-Bromo-4-chloro-3-indolyl octanoate, ci-après référencé X-C8,
- le 6-Chloro-3-indolyl octanoate, ci-après référencé Rose-C8, et
- le 5-Bromo-6-chloro-3-indolyl octanoate, ci-après référencé Magenta-C8.

Une solution mère de chaque substrat à 40 g/l est réalisée dans un mélange 40 % Diméthylsulfoxide et 60 % Tween 20. Un volume suffisant est ajouté à quatre milieux type Columbia en surfusion pour obtenir une concentration finale en substrat de 250 mg/l. Des micro-organismes issus de la collection de la demanderesse ou de la collection ATCC ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 2 ci-dessous :

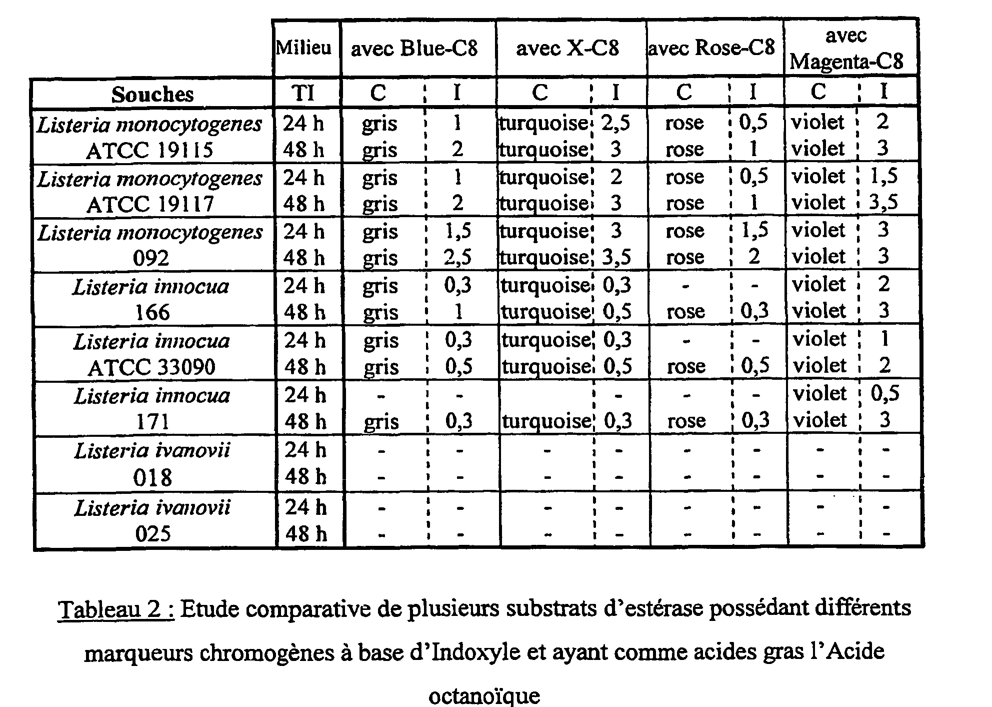

Quel que soit le substrat utilisé, l'écart d'intensité entre les souches de *Listeria monocytogenes* et *Listeria innocua* est conservé. Cet écart est cependant plus significatif pour les marqueurs 5-Bromo-4-chloro-3-indoxyle et 5-Bromo-3-indoxyle.

Le meilleur contraste et les intensités les plus fortes sont obtenus avec le marqueur donnant la coloration turquoise : le 5-Bromo-4-chloro-3-indoxyle.

### Expérience 3 : Détection d'une activité estérase dans un milieu liquide :

Les substrats chromogéniques d'estérase testés sont :
- le 5-Bromo-4-chloro-3-indolyl acétate, ci-après référencé X-C2,
- le 3-Indolyl acétate, ci-après référencé Y-C2,
- le 5-Bromo-4-chloro-3-indolyl butyrate, ci-après référencé X-C4,
- le 5-Bromo-4-chloro-3-indolyl octanoate, ci-après référencé X-C8,
- le, 5-Bromo-6-chloro-3-indolyl octanoate, ci-après référencé M-C8, et
- le 6-Chloro-3-indolyl octanoate, ci-après référencé R-C8.

Ces substrats ont été lyophilisés dans des cupules de type galeries API (marque déposée). Avant utilisation, les substrats contenus dans ces cupules sont remis en solution par l'apport d'un milieu d'inoculation type milieu Columbia sans agar. Des micro-organismes issus de la collection de la demanderesse ou de la collection ATCC ont été ensemencés dans ces cupules à partir d'une suspension à 2 McFarland. Les galeries ont été incubées à 37 °C pendant 8 heures. Les colonies formées ont été examinées visuellement après 4, 6 et 8 heures d'incubation.

L'intensité de coloration de ces colonies a été notée. Les résultats sont présentés dans le tableau 3 ci-dessous :

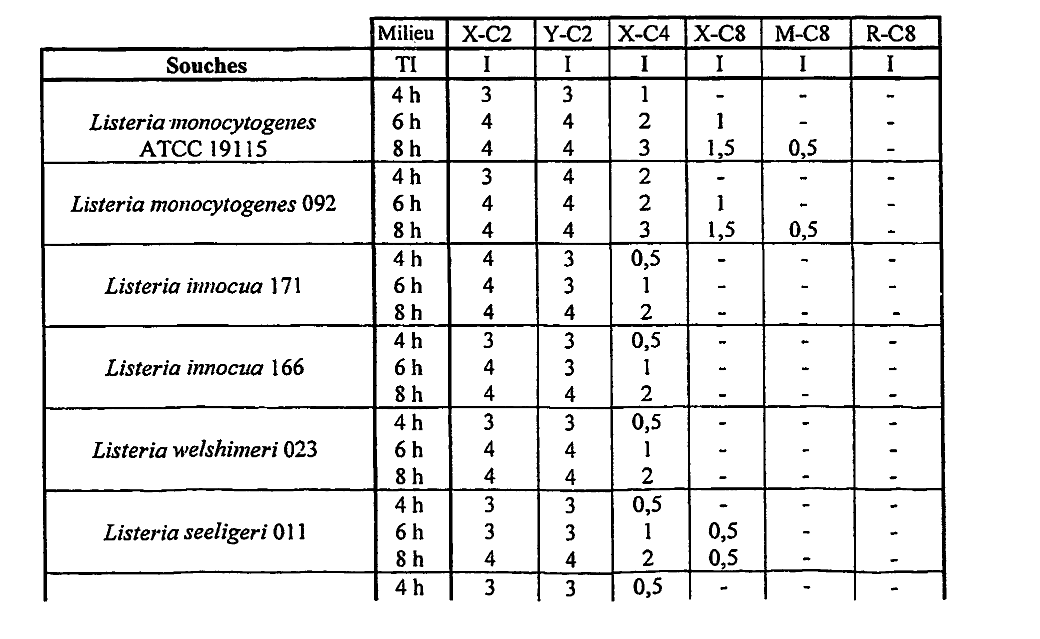

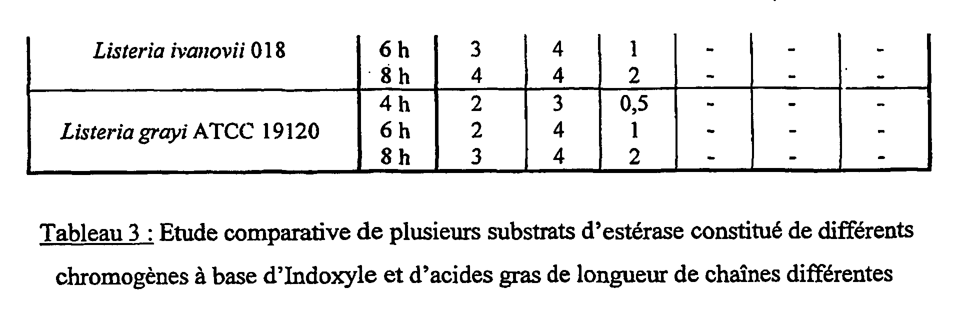

En milieu liquide, les substrats possédant une longueur de chaînes inférieures à 4 atomes de carbones ne sont pas discriminants pour différencier les différentes espèces de *Listeria* entre elles. Par contre, dans les conditions opératoires testées, les substrats X-C4 et X-C8 permettent d'obtenir un contraste de coloration entre *L. monocytogenes* d'une part, et les autres espèces du genre, d'autre part. En effet, seules *L. welshimeri* et *L. seeligeri* présentent aussi des colorations mais ces colorations sont très faibles. Il est possible d'imaginer « un seuil de positivité » du test égale à 1. Pour des intensités inférieures à 1, la souche testée serait considérée comme négative pour le test ; pour des intensités supérieures à 1, la souche testée serait positive. Donc dans le cas présent, il s'agirait d'une souche de *L. monocytogenes*.

### Expérience 4 : Détection simultanée d'une activité estérase et d'une activité osidase dans un milieu gélifié :

Différents couples de substrats chromogéniques d'estérase et de substrats chromogéniques d'osidase ont été testés, qui sont les suivants :
- 5-Bromo-3-indolyl octanoate et 6-Chloro-3-indolyl-β-D-glucoside, qui correspond au couple 1,
- 5-Bromo-3-indolyl octanoate et 6-Chloro-3-indolyl-β-D-cellobioside, correspondant au couple 2,
- 5-Bromo-3-indolyl octanoate et 6-Chloro-3-indolyl-N-acétyl-β-D-glucosaminide, qui correspond au couple 3,
- 5-Bromo-3-indolyl octanoate et 6-Chlore-3-indolyl-α-D-mannoside, correspondant au couple 4,
- 5-Bromo-4-chloro-3-indolyl nonanoate et 6-Chloro-3-indolyl-β-D-glucoside, qui correspond au couple 5,
- 5-Bromo-4-chloro-3-indolyl nonanoate et 6-Chloro-3-indolyl-β-D-cellobioside, correspondant au couple 6,
- 5-Bromo-4-chloro-3-indolyl nonanoate et 6-Chloro-3-indolyl-N-acétyl-β-D-glucosaminide, qui correspond au couple 7, et
- 5-Bromo-4-chloro-3-indolyl nonanoate et 6-Chloro-3-indolyl-α-D-mannoside, correspondant au couple 8.

Une solution mère de chaque substrat d'estérases à 40 g/l est réalisée dans un mélange 40% Diméthylsulfoxide et 60% Tween 20. Par ailleurs, une solution mère de chaque substrat d'osidases est réalisée dans du Diméthylsulfoxide. Un volume suffisant de la solution mère du substrat d'estérase, adapté à chacun des couples décrits ci-dessus, est ajouté à huit milieux type Columbia en surfusion, pour obtenir une concentration finale en substrat de 250 mg/l. Parallèlement, un volume suffisant du substrat d'osidases, adapté à chacun des couples décrits ci-dessus, est aussi ajouté à ces huit milieux, pour obtenir une concentration finale comprise entre 100 et 200 mg/l selon le substrat. Des micro-organismes issus de la collection de la demanderesse ou de la collection ATCC ont été ensemencés sur ce milieu par isolement en trois cadrans à partir d'une suspension à 0,5 McFarland. Les boîtes ont été incubées à 37°C pendant 48 heures. Les colonies formées ont été examinées visuellement après 24 et 48 heures d'incubation.

La coloration de ces colonies ainsi que l'intensité de cette coloration ont été notées. Les résultats sont présentés dans le tableau 4 ci-dessous :

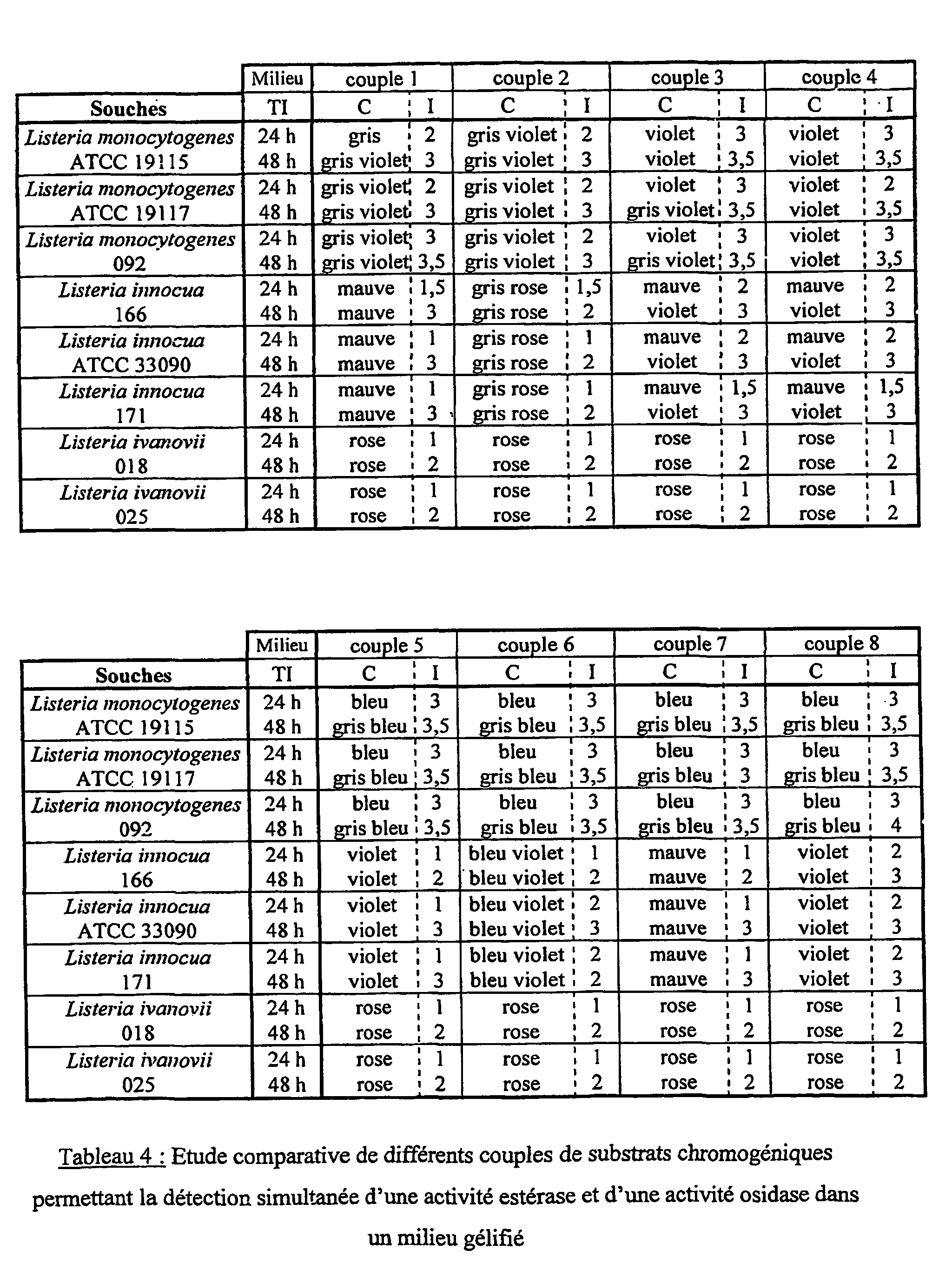

Quelques soient les couples de substrats étudiés, il y a une différence de coloration entre les souches de *Listeria monocytogenes* et les souches de *Listeria innocua* et *Listeria ivanovii*.

Le meilleur contraste est obtenu avec le 5-Bromo-4-chloro-3-indolyl nonanoate et un substrat d'osidase, quelle que soit l'activité cible, à base de 6-Chloro-3-indolyl. On peut donc coupler, *a priori,* tous les différents types de substrats d'estérase et d'osidase que ce soit des α-osidases ou des β-osidases.

Cependant, on peut observer que de meilleurs résultats sont obtenus avec le 6-Chloro-3-indolyl-β-D-glucoside, le 6-Chloro-3-indolyl-N-acétyl-β-D-glucosaminide et le 6-Chloro-3-indolyl-α-D-mannoside.

### Expérience 5 : Détection simultanée d'une activité estérase et d'une activité phosphatase dans un milieu gélifié :

Dans un milieu gélifié, type Columbia, en surfusion ont été ajoutées successivement deux solutions mères de deux substrats : le 5-Bromo-4-chloro-3-indolyl octanoate (X-C8) (concentration finale dans le milieu : 250 mg/l) et le 6-Chloro-3-indolyl phosphate (Rose P) (concentration finale dans le milieu 750 mg/l). La solution mère de 5-Bromo-4-chloro-3-indolyl octanoate a été préparée comme dans les exemples précédants et celle de 6-Chloro-3-indolyl phosphate a été réalisée à 50 g/l dans du Diméthylsulfoxide.

Des micro-organismes issus de la collection de la demanderesse ou de la collection ATCC ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation.

La coloration de ces colonies ainsi que l'intensité de cette coloration ont été notées. Les résultats sont présentés dans le tableau 5 ci-dessous :

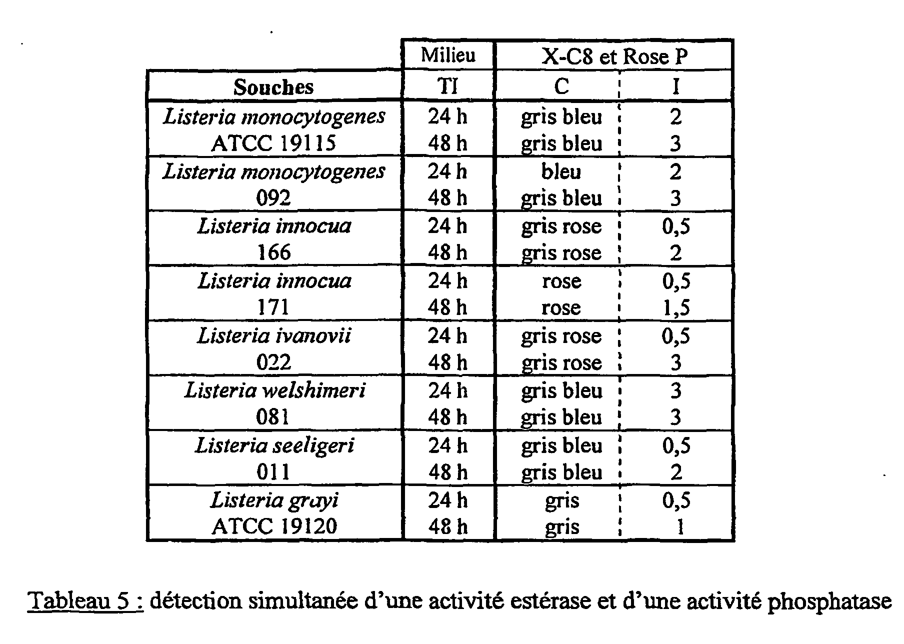

L'étude simultanée de ces deux activités permet la séparation de deux groupes de *Listeria*, d'une part, *L. monocytogenes, L. seeligeri* et *L. welshimeri* et d'autre part, *L. grayi, L. innocua* et *L. ivanovii*. Ce système de détection peut être amélioré en jouant sur la formulation du milieu, comme cela sera exposé dans l'expérience 6, où il est possible de séparer *L. monocytogenes* de toutes les autres espèces du genre *Listeria*.

### Expérience 6 : Identification de Listeria monocytogenes par rapport à Listeria welshimeri et Listeria seeligeri dans un milieu gélifié contenant un substrat d'estérase et un substrat d'osidase :

Le couple de substrats chromogéniques d'estérase et de substrats chromogéniques d'osidase testé est le suivant : 5-Bromo-4-chloro-3-indolyl octanoate et 6-Chloro-3-indolyl-N-acétyl-β-D-glucosaminide

La préparation des milieux et des solutions mères de substrat a été réalisée comme dans les exemples précédents. Ce milieu de base a été divisé en quatre milieux dans lesquels ont été ajoutés respectivement :
- 40 g/l de Xylose correspondant au milieu 1,
- 40 g/l de Tagatose correspondant au milieu 2,
- un mélange de 45 g/l d'Hydrates de carbone avec 30 g/l de Xylose et 15 g/l de Tagatose correspondant au milieu 3,
- un mélange de 65 g/l d'Hydrates de carbone avec 35 g/l de Xylose et 30 g/l de Tagatose correspondant au milieu 4.

Des micro-organismes issus de la collection de la demanderesse ont été ensemencés sur ces milieux par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation.

La coloration de ces colonies ainsi que l'intensité de cette coloration ont été notées. Les résultats sont présentés dans le tableau 6 ci-dessous :

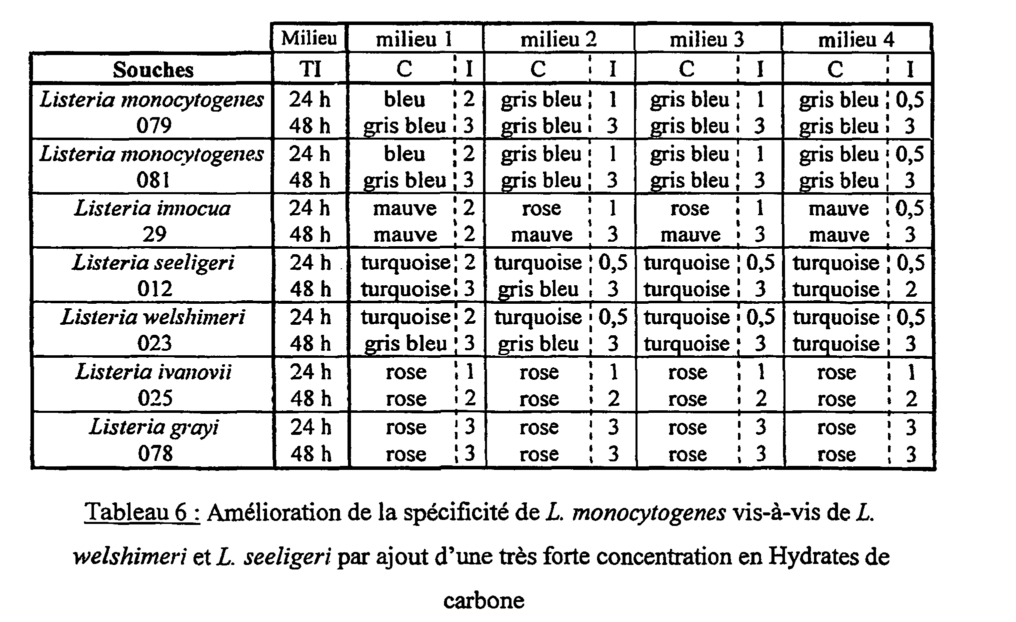

L'ajout d'Hydrates de carbone à fortes concentrations (supérieures ou égales à 40 g/l) permet la distinction de *L. monocytogenes* de toutes les autres espèces de *Listeria* et ceci uniquement à partir d'un critère de couleur. Néanmoins il a été démontré qu'il était possible d'obtenir une distinction entre espèces en utilisant des concentrations plus faibles pouvant aller jusqu'à 10 g/l, comme le prouve le contenu du brevet FR-B-2.708.285. Cependant à la différence de ce brevet, nous avons un changement de couleur par inhibition d'une activité enzymatique, et non une couleur dérivée différente de la couleur de base du marqueur.

### Expérience 7 : Addition d'agents sélectifs pour inhiber les bactéries à Gram positif autres que Listeria et les bactéries à Gram négatif ainsi que les levures :

Le milieu de base type Columbia contient :
- du 5-Bromo-4-chloro-3-indolyl octanoate à 250 mg/l apporté par une solution mère dans un mélange 40 % Diméthylsulfoxide et 60 % Polyoxyéthylènesorbitan Monolaurate (Tween 20),
- du 5-Bromo-6-chloro-3-indolyl-β-N-acétylglucosaminide à 150 mg/l apporté par une solution mère dans le DMSO,
- du Xylose à 10 g/l et du Tagatose à 5 g/l, et
- un mélange sélectif défini comme suit : Chlorure de Lithium à 5 g/l, Ceftazidime à 20 mg/l, Amphotéricine B à 4 mg/l, Fosfomycine à 10 mg/l et Colistine à 5 mg/l.

Des micro-organismes issus de la collection de la demanderesse ont été ensemencés sur ce milieu par isolement en trois cadrans à partir d'une suspension à 0,5 McFarland. Les boîtes ont été incubées à 37 °C pendant 48 heures. Les colonies formées ont été examinées visuellement après 24 et 48 heures d'incubation. La coloration de ces colonies ainsi que l'intensité de cette coloration ont été notées. Les résultats sont présentés dans le tableau 7 ci-dessous :

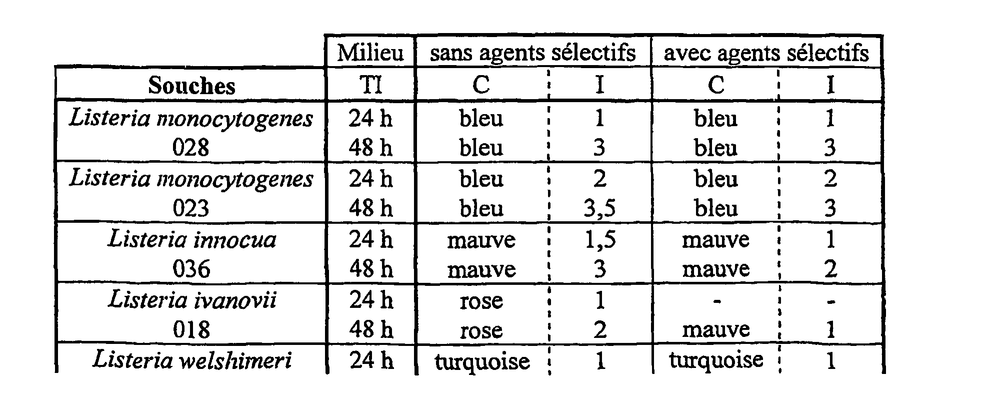

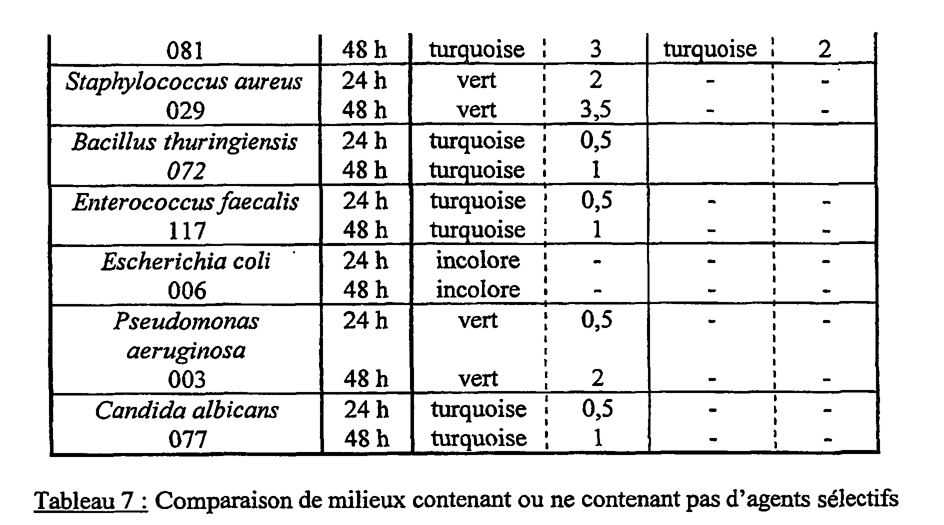

Dans le tableau 7 ci-dessus, C représente la couleur des colonies après incubation, I représente l'intensité de cette coloration, le symbole « - » est synonyme d'une absence de croissance, enfin TI définit les temps d'incubation. A noter que l'intensité de la coloration est une échelle arbitraire, mais qui est commune à l'ensemble des échantillons biologiques et des milieux testés.

Il est possible de remarquer, qu'en présence d'agents sélectifs la totalité des bactéries et levures interférentes testées (c'est-à-dire non *Listeria*) ne présentent pas de croissance et sont donc inhibées. La présence d'agents sélectifs ne modifie pas les couleurs des souches de *Listeria* testées.

Cette expérience montre également que, même sans agents sélectifs, la différenciation entre *Listeria monocytogenes*, d'une part, et les bactéries et levures interférentes testées, c'est-à-dire incluant les autres *Listeria*, d'autre part, est possible, ce qui n'est pas le cas avec les substrats et les milieux de culture ou réactionnels décrits dans l'état de la technique.

## Revendications

1. Utilisation d'un substrat pour l'identification directe de bactéries pathogènes *Listeria monocytogenes*, **caractérisé par le fait que** le substrat est clivé par une activité estérase, différente de l'activité phospholipase C, spécifique du Phosphatidylinositol (PI-PLC), activité estérase qui est spécifique de *Listeria monocytogenes* et qu'il comprend une partie cible constituée par un acide gras ayant une chaîne carbonée de longueur comprise entre 4 et 20 atomes de carbones.

2. Utilisation d'un substrat, selon la revendication 1, **caractérisé par le fait que** l'activité estérase est une activité enzymatique spécifique, c'est-à-dire qui clive, la liaison ester entre la partie marqueur et la partie cible qui constituent le substrat.

3. Utilisation d'un substrat, selon la revendication 2, **caractérisé par le fait que** la liaison clivée est une liaison ester entre une fonction alcool portée par la partie marqueur, et un acide organique constituant la partie cible.

4. Utilisation d'un substrat, selon la revendication 3, **caractérisé par le fait que** la partie marqueur est constituée par un chromogène, tel qu'un Indoxyle pouvant être constitué par l'un des constituants suivants
• 5-Bromo-3-indoxyle, ou
• 5-Bromo-4-chloro-3-indoxyle, ou
• 6-Chloro-3-indoxyle, ou
• 5-Bromo-6-chloro-3-indoxyle, ou
• 6-Bromo-3-indoxyle.

5. Utilisation d'un substrat, selon la revendication 1, **caractérisé par le fait que** la partie cible est constituée par un acide gras ayant un chaîne carbonée de longueur comprise entre 4 et 10 atomes de carbones.

6. Utilisation d'un substrat, selon la revendication 4, **caractérisé par** le fait qu'il est constitué par le 5-Bromo-4-chloro-3-indolyl butyrate, le 5-Bromo-4-chloro-3-indolyl octanoate, 5-Bromo-4-chloro-3-indolyl nonanoate ou le 5-Bromo-4-chloro-3-indolyl décanoate.

7. Utilisation d'un substrat, selon l'une quelconque des revendications 1 à 6, **caractérisé par le fait que** le substrat est couplé à au moins un autre substrat permettant de détecter au moins une autre activité enzymatique possédée par toute ou partie des espèces de *Listeria.*

8. Utilisation de substrats, selon la revendication 7, **caractérisés par le fait que** le substrat permettant de détecter l'activité estérase, à l'exception de l'enzyme PI-PLC, a une coloration différente de celle de l'autre substrat permettant de détecter une activité enzymatique différente de l'activité estérase précédemment définie.

9. Utilisation de substrats, selon l'une quelconque des revendications 7 ou 8, **caractérisés par le fait que** l'autre activité enzymatique possédée par toute ou partie des espèces de *Listeria* est une activité osidase, phosphatase ou aminopeptidase.

10. Utilisation de substrats, selon l'une quelconque des revendications 7 à 9, **caractérisés par le fait que** la partie marqueur de l'autre substrat est constituée à base de:
• 5-Bromo-3-indoxyle, ou
• 5-Bromo-4-chloro-3-indoxyle, ou
• 6-Chloro-3-indoxyle, ou
• 5-Bromo-6-chloro-3-indoxyle, ou
• 6-Bromo-3-indoxyle.

11. Utilisation de substrats, selon la revendication 10, **caractérisés par le fait que** l'autre substrat est constitué par
• 5-Bromo-6-chloro-3-indolyl-β-D-glucoside, ou
• 6-Chloro-3-indolyl-β-D-glucoside, ou
• 6-Chloro-3-indolyl-β-D-cellobioside, ou
• 6-Chloro-3-indolyl-N-acétyl-β-D-glucosaminide, ou
• 6-Chloro-3-indolyl-α-D-mannoside, ou
• 6-Chloro-3-indolylphosphate.

12. Utilisation d'un milieu réactionnel pour l'identification directe de *Listeria monocytogenes,* qui utilise au moins un substrat clivé par une activité estérase, différente de l'activité phospholipase C, spécifique du Phosphatidylinositol (PI-PLC), activité estérase qui est spécifique de *Listeria monocytogenes* et qu'il comprend une partie cible constituée par un acide gras ayant une chaîne carbonée de longueur comprise entre 4 et 20 atomes de carbones.

13. Utilisation d'un milieu, selon la revendication 12, **caractérisé par le fait que** le substrat permettant de détecter une activité estérase, différente de l'activité PI-PLC, a une concentration comprise entre 20 mg/l et 3 g/l, ou préférentiellement entre 50 mg/l et 1 g/l, ou préférentiellement entre 100 et 600 mg/l, ou préférentiellement environ de 250 mg/l.

14. Utilisation d'un milieu, selon l'une quelconque des revendications 12 ou 13, **caractérisé par** le fait qu'il comprend au moins un autre substrat permettant de détecter une autre activité, telle qu'une activité osidase, phosphatase ou aminopeptidase, a une concentration comprise entre 10 mg/l et 500 mg/l, préférentiellement entre 50 et 300 mg/l, et encore plus préférentiellement entre 100 et 200 mg/l.

15. Utilisation d'un milieu, selon l'une quelconque des revendications 12 à 14, **caractérisé par le fait que** le milieu est un milieu gélosé ou liquide.

16. Utilisation d'un milieu, selon l'une quelconque des revendications 12 à 15, **caractérisé par le fait qu'**il comporte un moyen permettant de discriminer *Listeria monocytogenes* par rapport à *Listeria welshimeri* et *Listeria seeligeri,* à savoir :
• ajout d'au moins un Hydrate de carbone acidifié (par exemple Xylose et/ou D-Tagatose) par *Listeria welshimeri* et *Listeria seeligeri* et pas par *Listeria monocytogenes*, ou
• ajout d'au moins un Hydrate de carbone acidifié par *Listeria monocytogenes* et éventuellement *Listeria innocua* et/ou *Listeria ivanovii* et/ou *Listeria grayii* ; ou
• ajout d'au moins un substrat permettant de révéler une activité osidase ((β-maltosidase) et/ou phosphatase et/ou aminopeptidase (L-glycine aminopeptidase) spécifique(s) au moins de *Listeria welshimeri* et *Listeria seeligeri* mais pas de *Listeria monocytogenes*, ou
• ajout d'au moins un substrat naturel de Phospholipase (PLC), tel que Phosphatidylinositol (PI) et/ou Phosphatidylcholine (PC).

17. Utilisation d'un milieu, selon l'une quelconque des revendications 12 à 16, **caractérisé par** le fait qu'il comporte un moyen sélectif permettant de discriminer *Listeria monocytogenes* par rapport à au moins les espèces suivantes:
• *Staphylococcus aureus ;*
• *Bacillus thuringiensis ;*
• *Enterococcus faecalis ;*
• *Escherichia coli ;*
• *Pseudomonas aeruginosa ;* et
• *Candida albicans.*

18. Utilisation d'un milieu, selon la revendication 17, **caractérisé par le fait que** le moyen sélectif est constitué par au moins l'un des composés suivants :
• Chlorure de Lithium ;
• Ceftazidime ;
• Amphotéricine B ;
• Fosfomycine ; et/ou
• Colistine.

19. Procédé d'identification de bactéries pathogènes de l'espèce *Listeria monocytogenes*, comprenant
• l'ensemencement d'un échantillon susceptible de contenir les bactéries pathogènes sur un milieu de culture, qui utilise au moins un substrat clivé par une activité estérase, différente de l'activité phospholipase C, spécifique du Phosphatidylinositol (PI-PLC), activité estérase qui est spécifique de *Listeria monocytogenes* et qu'il comprend une partie cible constituée par un acide gras ayant une chaîne carbonée de longueur comprise entre 4 et 20 atomes de carbones ;
• l'incubation du milieu de culture ensemencé avec l'échantillon, et
• la détermination de la présence desdites bactéries pathogènes par la couleur et l'intensité caractéristiques du ou des substrats.

20. Procédé, selon la revendication 19, **caractérisé en ce que** ledit échantillon, susceptible de contenir des bactéries pathogènes est préalablement enrichi avant d'être ensemencé sur un milieu de culture, qui utilise au moins un substrat clivé par une activité estérase, différente de l'activité phospholipase C, spécifique du Phosphatidylinositol (PI-PLC), activité estérase qui est spécifique de *Listeria monocytogenes* et qu'il comprend une partie cible constituée par un acide gras ayant une chaîne carbonée de longueur comprise entre 4 et 20 atomes de carbones.

21. Procédé, selon l'une quelconque des revendications 19 ou 20, **caractérisé en ce que** l'on effectue la détermination de la présence desdites bactéries pathogènes, par la couleur et l'intensité caractéristiques du ou des substrats, après 18 à 24 heures d'incubation.

22. Utilisation in vitro d'un substrat clivé par une activité estérase, différente de l'activité phospholipase C, spécifique du Phosphatidylinositol (PI-PLC), activité estérase qui est spécifique de *Listeria monocytogenes* et qui comprend une partie cible constituée par un acide gras ayant une chaîne carbonée de longueur comprise entre 4 et 20 atomes de carbones, et d'une partie inhibitrice pour inhiber spécifiquement *Listeria monocytogenes* lorsque la partie inhibitrice est libérée.

## Patentansprüche

1. Eine Verwendung eines Substrats zum direkten Nachweis der pathogenen Bakterien *Listeria monocytogenes*, **dadurch gekennzeichnet, dass** das Substrat durch eine Esteraseaktivität, die sich von der für Phosphatidylinositol (PI-PLC) spezifischen Phospholipase-C-Aktivität unterscheidet, gespalten wird, wobei die Esteraseaktivität für *Listeria monocytogenes* spezifisch ist, und das einen Zielteil umfasst, der aus einer Fettsäure mit einer Kohlenstoffkette einer Länge von 4 bis 20 Kohlenstoffatomen zusammengesetzt ist.

2. Verwendung eines Substrats gemäß Anspruch 1, **dadurch gekennzeichnet, dass** die Esteraseaktivität eine spezifische Enzymaktivität ist, das heißt, sie spaltet die Esterbindung zwischen dem Markerteil und dem Zielteil, die das Substrat bilden.

3. Verwendung eines Substrats gemäß Anspruch 2, **dadurch gekennzeichnet, dass** die gespaltene Bindung eine Esterbindung zwischen einer vom Markerteil getragenen Alkoholfunktion und einer den Zielteil bildenden organischen Säure ist.

4. Verwendung eines Substrats gemäß Anspruch 3, **dadurch gekennzeichnet, dass** der Markerteil aus einem Chromogen zusammengesetzt ist, wie etwa ein Indoxyl, das aus einem der folgenden Bestandteile zusammengesetzt sein kann:
• 5-Brom-3-indoxyl oder
• 5-Brom-4-chlor-3-indoxyl oder
• 6-Chlor-3-indoxyl oder
• 5-Brom-6-chlor-3-indoxyl oder
• 6-Brom-3-indoxyl.

5. Verwendung eines Substrats gemäß Anspruch 1, **dadurch gekennzeichnet, dass** der Zielteil aus einer Fettsäure mit einer Kohlenstoffkette einer Länge von 4 bis 10 Kohlenstoffatomen zusammengesetzt ist.

6. Verwendung eines Substrats gemäß Anspruch 4, **dadurch gekennzeichnet, dass** es aus 5-Brom-4-chlor-3-indolylbutyrat, 5-Brom-4-chlor-3-indolyloctanoat, 5-Brom-4-chlor-3-indolylnonanoat oder 5-Brom-4-chlor-3-indolyldecanoat zusammengesetzt ist.

7. Verwendung eines Substrats gemäß einem der Ansprüche 1 bis 6, **dadurch gekennzeichnet, dass** das Substrat mit mindestens einem anderen Substrat gekoppelt ist, das das Ermitteln mindestens einer anderen Enzymaktivität der gesamten oder eines Teils der Spezies *Listeria* ermöglicht.

8. Verwendung von Substraten gemäß Anspruch 7, **dadurch gekennzeichnet, dass** das Substrat, das das Ermitteln der Esteraseaktivität, mit Ausnahme des Enzyms PI-PLC, ermöglicht, eine andere Färbung aufweist als das andere Substrat, was das Ermitteln einer anderen Enzymaktivität als die vorangehend definierte Esteraseaktivität ermöglicht.

9. Verwendung von Substraten gemäß Anspruch 7 oder 8, **dadurch gekennzeichnet, dass** die andere Enzymaktivität der gesamten oder eines Teil der Spezies *Listeria* eine Osidase-, Phosphatase- oder Aminopeptidaseaktivität ist.

10. Verwendung von Substraten gemäß einem der Ansprüche 7 bis 9, **dadurch gekennzeichnet, dass** der Markerteil des anderen Substrats aus Folgendem als Basis zusammengesetzt ist:
• 5-Brom-3-indoxyl oder
• 5-Brom-4-chlor-3-indoxyl oder
• 6-Chlor-3-indoxyl oder
• 5-Brom-6-chlor-3-indoxyl oder
• 6-Brom-3-indoxyl.

11. Verwendung von Substraten gemäß Anspruch 10, **dadurch gekennzeichnet, dass** das andere Substrat aus Folgendem zusammengesetzt ist:
• 5-Brom-6-chlor-3-indolyl-β-D-glucosid oder
• 6-Chlor-3-indolyl-β-D-glucosid oder
• 6-Chlor-3-indolyl-β-D-cellobiosid oder
• 6-Chlor-3-indolyl-N-acetyl-β-D-glucosaminid oder
• 6-Chlor-3-indolyl-α-D-mannosid oder
• 6-Chlor-3-indolylphosphat.

12. Eine Verwendung eines Reaktionsmediums zum direkten Nachweis von *Listeria monocytogenes*, die mindestens ein Substrat verwendet, das durch eine Esteraseaktivität, die sich von der für Phosphatidylinositol (PI-PLC) spezifischen Phospholipase-C-Aktivität unterscheidet, gespalten wird, wobei die Esteraseaktivität spezifisch für *Listeria monocytogenes* ist, und die einen Zielteil umfasst, der aus einer Fettsäure mit einer Kohlenstoffkette einer Länge von 4 bis 20 Kohlenstoffatomen zusammengesetzt ist.

13. Verwendung eines Mediums gemäß Anspruch 12, **dadurch gekennzeichnet, dass** das Substrat, das das Ermitteln einer sich von der PI-PLC-Aktivität unterscheidenden Esteraseaktivität ermöglicht, eine Konzentration von 20 mg/l bis 3 g/l, oder vorzugsweise von 50 mg/l bis 1 g/l, oder vorzugsweise von 100 bis 600 mg/l oder vorzugsweise von ungefähr 250 mg/l aufweist.

14. Verwendung eines Mediums gemäß Anspruch 12 oder 13, **dadurch gekennzeichnet, dass** es mindestens ein anderes Substrat umfasst, das das Ermitteln einer anderen Aktivität, wie etwa einer Osidase-, Phosphatase oder Aminopeptidaseaktivität, ermöglicht, eine Konzentration von 10 mg/l bis 500 mg/l, vorzugsweise von 50 bis 300 mg/l und am besten von 100 bis 200 mg/l aufweist.

15. Verwendung eines Mediums gemäß einem der Ansprüche 12 bis 14, **dadurch gekennzeichnet, dass** das Medium ein Agar-Agar-Medium oder ein flüssiges Medium ist.

16. Verwendung eines Mediums gemäß einem der Ansprüche 12 bis 15, **dadurch gekennzeichnet, dass** es ein Mittel beinhaltet, das es ermöglicht, *Listeria monocytogenes* von *Listeria welshimeri* und *Listeria seeligeri* zu unterscheiden, nämlich:
• Zugabe mindestens eines durch *Listeria welshimeri* und *Listeria seeligeri* und nicht durch *Listeria monocytogenes* azidifizierten Kohlenhydrats (zum Beispiel Xylose und/oder D-Tagatose) oder
• Zugabe mindestens eines durch *Listeria monocytogenes* und eventuell *Listeria innocua* und/oder *Listeria ivanovii* und/oder *Listeria grayii* azidifizierten Kohlenhydrats oder
• Zugabe mindestens eines Substrats, das das Aufdecken einer für mindestens *Listeria welshimeri* und *Listeria seeligeri* jedoch nicht für *Listeria monocytogenes* spezifischen Osidase-(β-Maltosidase) und/oder Phosphatase- und/oder Aminopeptidase-(L-glycinaminopeptidase)-Aktivität ermöglicht, oder
• Zugabe mindestens eines natürlichen Substrats aus Phospholipase (PLC), wie etwa Phosphatidylinositol (PI) und/oder Phosphatidylcholin (PC).

17. Verwendung eines Mediums gemäß einem der Ansprüche 12 bis 16, **dadurch gekennzeichnet, dass** es ein selektives Mittel beinhaltet, das es ermöglicht, *Listeria monocytogenes* von mindestens den folgenden Spezies zu unterscheiden:
• *Staphlyococcus aureus,*
• *Bacillus thuringiensis,*
• *Enterococcus faecalis,*
• *Escherichia coli,*
• *Pseudomonas aeruginosa* und
• *Candida albicans*.

18. Verwendung eines Mediums gemäß Anspruch 17, **dadurch gekennzeichnet, dass** das selektive Mittel aus mindestens einem der folgenden Bestandteile zusammengesetzt ist:
• Lithiumchlorid,
• Ceftazidim,
• Amphoterizin B,
• Fosfomyzin und/oder
• Colistin.

19. Ein Verfahren zum Nachweis von pathogenen Bakterien der Spezies *Listeria monocytogenes*, das Folgendes umfasst:
• Beimpfung eines Kulturmediums mit einer Probe, die die pathogenen Bakterien enthalten kann, das mindestens ein Substrat verwendet, das durch eine Esteraseaktivität, die sich von der für Phosphatidylinositol (PI-PLC) spezifischen Phospholipase-C-Aktivität unterscheidet, gespalten wird, wobei die Esteraseaktivität spezifisch für *Listeria monocytogenes* ist, und das einen Zielteil umfasst, der aus einer Fettsäure mit einer Kohlenstoffkette einer Länge von 4 bis 20 Kohlenstoffatomen zusammengesetzt ist;
• Inkubation des mit der Probe beimpften Kulurmediums und
• Bestimmung der Anwesenheit der pathogenen Bakterien anhand der charakteristischen Farbe und Intensität des Substrats oder der Substrate.

20. Verfahren gemäß Anspruch 19, **dadurch gekennzeichnet, dass** die Probe, die pathogene Bakterien enthalten kann, vor dem Beimpfen eines Kulturmediums angereichert wird, das mindestens ein Substrat verwendet, das durch eine Esteraseaktivität, die sich von der für Phosphatidylinositol (PI-PLC) spezifischen Phospholipase-C-Aktivität unterscheidet, gespalten wird, wobei die Esteraseaktivität spezifisch für *Listeria monocytogenes* ist, und das einen Zielteil umfasst, der aus einer Fettsäure mit einer Kohlenstoffkette einer Länge von 4 bis 20 Kohlenstoffatomen zusammengesetzt ist.

21. Verfahren gemäß Anspruch 19 oder 20, **dadurch gekennzeichnet, dass** nach 18 bis 24 Stunden Inkubation anhand der charakteristischen Farbe und Intensität des Substrats oder der Substrate die Bestimmung der Anwesenheit der pathogenen Bakterien vorgenommen wird.

22. Die In-vitro-Verwendung eines Substrats, das durch eine Esteraseaktivität, die sich von der für Phosphatidylinositol (PI-PLC) spezifischen Phospholipase-C-Aktivität unterscheidet, gespalten wird, wobei die Esteraseaktivität spezifisch für *Listeria monocytogenes* ist, und das einen Zielteil, der aus einer Fettsäure mit einer Kohlenstoffkette einer Länge von 4 bis 20 Kohlenstoffatomen zusammengesetzt ist, und einen hemmenden Teil zur spezifischen Hemmung von *Listeria monocytogenes* während der Freisetzung des hemmenden Teils umfasst.

## Claims

1. Use of a substrate for the direct identification of *Listeria monocytogenes* pathogenic bacteria, **characterised in that** the substrate is split by an esterase activity, differing from the phospholipase C activity, specific to Phosphatidylinositol (PI-PLC), which esterase activity is specific to *Listeria monocytogenes*, and that it includes a target part constituted by a fatty acid having a carbon chain with a length of between 4 and 20 carbon atoms.

2. The use of a substrate, according to Claim 1, **characterised in that** the esterase activity is a specific enzymatic activity, i.e. which splits the ester bond between the marker part and the target part which constitute the substrate.

3. The use of a substrate, according to Claim 2, **characterised in that** the split bond is an ester bond between an alcohol function carried by the marker part and an organic acid constituting the target part.

4. The use of a substrate, according to Claim 3, **characterised in that** the marker part is constituted by a chromogen, such as an Indoxyl which can be constituted by one of the following constituents
• 5-Bromo-3-indoxyl, or
• 5-Bromo-4-chloro-3-indoxyl, or
• 6-Chloro-3-indoxyl, or
• 5-Bromo-6-chloro-3-indoxyl, or
• 6-Bromo-3-indoxyl.

5. The use of a substrate, according to Claim 1, **characterised in that** the target part is constituted by a fatty acid having a carbon chain with a length of between 4 and 10 carbon atoms.

6. The use of a substrate, according to Claim 4, **characterised in that** it is constituted by 5-Bromo-4-chloro-3-indolyl butyrate, 5-Bromo-4-chloro-3-indolyl octanoate, 5-Bromo-4-chloro-3-indolyl nonanoate or 5-Bromo-4-chloro-3-indolyl decanoate.

7. The use of a substrate, according to any one of Claims 1 to 6, **characterised in that** the substrate is coupled to at least one other substrate enabling the detection of at least one other enzymatic activity which all or part of the *Listeria* species has.

8. The use of substrates, according to Claim 7, **characterised in that** the substrate enabling the detection of the esterase activity, with the exception of the PI-PLC enzyme, has a different colouring to that of the other substrate enabling the detection of an enzymatic activity which differs from the esterase activity previously described.

9. The use of substrates, according to any one of Claims 7 or 8, **characterised in that** the other enzymatic activity which all or part of the *Listeria* species has is an osidase, phosphatase or aminopeptidase activity.

10. The use of substrates, according to any one of Claims 7 to 9, **characterised in that** the marker part of the other substrate is constituted with, as a base:
• 5-Bromo-3-indoxyl, or
• 5-Bromo-4-chloro-3-indoxyl, or
• 6-Chloro-3-indoxyl, or
• 5-Bromo-6-chloro-3-indoxyl, or
• 6-Bromo-3-indoxyl.

11. The use of substrates, according to Claim 10, **characterised in that** the other substrate is constituted by
• 5-Bromo-6-chloro-3-indolyl-β-D-glucoside, or
• 6-Chloro-3-indolyl-β-D-glucoside, or
• 6-Chloro-3-indolyl-β-D-cellobioside, or
• 6-Chloro-3-indolyl-N-acetyl-β-D-glucosaminide, or
• 6-Chloro-3-indolyl-α-D-mannoside, or
• 6-Chloro-3-indolylphosphate.

12. Use of a reaction medium for the direct identification of *Listeria monocytogenes* which uses at least one substrate split by an esterase activity, differing from the phospholipase C activity, specific to Phosphatidylinositol (PI-PLC), which esterase activity is specific to *Listeria monocytogenes*, and which includes a target part constituted by a fatty acid having a carbon chain with a length of between 4 and 20 carbon atoms.

13. The use of a medium, according to Claim 12, **characterised in that** the substrate enabling the detection of an esterase activity differing from the PI-PLC activity has a concentration of between 20mg/l and 3g/l, or preferably of between 50mg/l and 1g/l, or preferably of between 100 and 600mg/l, or preferably of around 250mg/l.

14. The use of a medium, according to any one of Claims 12 or 13, **characterised in that** it includes at least one other substrate enabling the detection of one other activity, such as an osidase, phosphatase or aminopeptidase activity, having a concentration of between 10mg/l and 500mg/l, preferably of between 50 and 300mg/l, and still more preferably of between 100 and 200mg/l.

15. The use of a medium, according to any one of Claims 12 to 14, **characterised in that** the medium is an agar or a liquid medium.

16. The use of a medium, according to any one of Claims 12 to 15, **characterised in that** it comprises a means enabling *Listeria monocytogenes* to be distinguished from *Listeria weishimeri* and *Listeria seeligeri,* namely:
• addition of at least one Carbohydrate (for example Xylose and/or D-Tagatose) acidified by *Listeria welshimeri* and *Listeria seeligeri* and not by *Listeria monocytogenes*, or
• addition of at least one Carbohydrate acidified by *Listeria monocytogenes* and possibly *Listeria innocua* and/or *Listeria ivanovii* and/or *Listeria grayii;* or
• addition of at least one substrate enabling the revealing of an osidase (β-maltosidase) and/or phosphatase and/or aminopeptidase (L-glycine aminopeptidase) activity specific at least to *Listeria weishimeri* and *Listeria seeligeri* but not to *Listeria monocytogenes*, or
• addition of at least one natural Phospholipase substrate (PLC), such as Phosphatidylinositol (PI) and/or Phosphatidylcholine (PC).

17. The use of a medium, according to any one of Claims 12 to 16, **characterised in that** it comprises a selective means enabling *Listeria monocytogenes* to be distinguished from at least the following species:
• *Staphlyococcus aureus;*
• *Bacillus thuringiensis;*
• *Enterococcus faecalis;*
• *Escherichia coli;*
• *Pseudomonas aeruginosa;* and
• *Candida albicans*.

18. The use of a medium, according to Claim 17, **characterised in that** the selective means is constituted by at least one of the following compounds:
• Lithium Chloride;
• Ceftazidime;
• Amphotericin B;
• Fosfomycin; and/or
• Colistine.

19. A method for identifying pathogenic bacteria of the *Listeria monocytogenes* species, including
• seeding a sample which may contain the pathogenic bacteria on a culture medium, which uses at least one substrate split by an esterase activity, differing from the phospholipase C activity, specific to Phosphatidylinositol (PI-PLC), which esterase activity is specific to *Listeria monocytogenes*, and which includes a target part constituted by a fatty acid having a carbon chain with a length of between 4 and 20 carbon atoms;
• incubating the seeded culture medium with the sample, and
• determining the presence of said pathogenic bacteria by the characteristic colour and intensity of the substrate(s).

20. The method, according to Claim 19, **characterised in that** said sample which may contain pathogenic bacteria is pre-enriched before being seeded on a culture medium, which uses at least one substrate split by an esterase activity, differing from the phospholipase C activity, specific to Phosphatidylinositol (PI-PLC), which esterase activity is specific to *Listeria monocytogenes*, and which includes a target part constituted by a fatty acid having a carbon chain with a length of between 4 and 20 carbon atoms;

21. The method, according to any one of Claims 19 or 20, **characterised in that** the determination of the presence of said pathogenic bacteria, by the characteristic colour and intensity of the substrate(s), is effected after 18 to 24 hours of incubation.

22. In vitro use of a substrate split by an esterase activity, differing from the phospholipase C activity, specific to Phosphatidylinositol (PI-PLC), which esterase activity is specific to *Listeria monocytogenes*, and which includes a target part constituted by a fatty acid having a carbon chain with a length of between 4 and 20 carbon atoms, and an inhibiting part for specifically inhibiting *Listeria monocytogenes* when the inhibiting part is freed.
